Europäisches Patentamt

European Patent Office

Office européen des brevets

(11) Publication number: **0 075 293**
**B1**

# EUROPEAN PATENT SPECIFICATION

(45) Date of publication of patent specification: **03.09.86**

(21) Application number: **82108604.8**

(22) Date of filing: **17.09.82**

(51) Int. Cl.⁴: **C 12 N 9/99, C 12 Q 1/34** // C12N9/38

(54) Method for inhibiting beta-D-galactosidase.

(30) Priority: **18.09.81 JP 146394/81**

(43) Date of publication of application:
**30.03.83 Bulletin 83/13**

(45) Publication of the grant of the patent:
**03.09.86 Bulletin 86/36**

(84) Designated Contracting States:
**DE FR NL**

(56) References cited:
**GB-A-2 102 946**

STEROIDS, vol. 37, no. 3, March 1981, pages
303-314, San Francisco, California, USA G.
KOMINAMI et al.: "An enzyme immunosssay
for plasma betamethasone"

R.J. MAYER, J.H. WALKER: "Immunochemical
methods in the biological sciences: enzymes
and proteins" 1980, Academic Press, New York,
USA

PATENTS ABSTRACTS OF JAPAN, vol. 6, no.
70, 6th May 1982, page 948 P 113

H.R. MAHLER & EUGENE H. CORDES:
"Biological chemistry", 1966, Harper
International, New York, USA

(73) Proprietor: **AJINOMOTO CO., INC.**
5-8, Kyobashi 1-chome, Chuo-ku
Tokyo 104 (JP)

(73) Proprietor: **FUJIREBIO KABUSHIKI KAISHA also
trading as FUJIREBIO INC.**
6-7, Shimoochiai 4-chome Shinjuku-ku
Tokyo 161 (JP)

(72) Inventor: **Takenouchi, Tomoharu**
No. 2-15-3, Nakamachi
Setagaya-ku Tokyo (JP)
Inventor: **Kamimura, Akira**
No. 2-25-19, Ikego
Zushi-shi Kanagawa-ken (JP)

(74) Representative: **Schübel-Hopf, Ursula et al**
Strehl, Schübel-Hopf, Schulz Patentanwälte
Widenmayerstrasse 17
D-8000 München 22 (DE)

(56) References cited:

BIOCHEMISTRY, vol. 18, no. 19, 1979, pages
4090-4095, Easton, Pa., USA R.E. HUBER et al.:
"Interaction of divalent cations with beta-
galactosidase (Escherichia coli)"

Courier Press, Leamington Spa, England.

(56) References cited:

CHEMICAL ABSTRACTS, vol. 79, no. 11, 17th
September 1973, page 184, no. 63185r,
Columbus, Ohio, USA G.S. CASE et al.: "Role of
magnesium ions in beta-galactosidase-
catalyzed hydrolyses. Charge and shape of the
beta-galactopyranosyl binding site"

CHEMICAL ABSTRACTS, vol. 73, no. 11, 14th
September 1970, page 27, no. 52487r,
Columbus, Ohio, USA S. SHIFRIN et al.:
"Inactivation and dissociation of beta-
galactosidase by amines"

CHEMICAL ABSTRACTS, vol. 91, no. 25, 17th
December 1979, page 492, no. 209054u,
Columbus, Ohio, USA E. ISHIKAWA et al.:
"Improved procedure for the preparation ogf
antibody-beta-D-galactosidase conjugates"

## Description

The present invention relates to a novel method for inhibiting β-D-galactosidase in order to terminate β-D-galactosidase catalyzed reactions.

In recent years, β-D-galactosidase has progressively found its application as an enzyme for labelling an antibody or an antigen in enzyme immunoassays (hereinafter referred to as "EIA"): In EIA, since the measured value of the β-D-Galactosidase activity directly reflects the amount of the component to be measured, it is necessary, on measuring the β-D-galactosidase activity, to obtain the accurate measured value of the enzyme reaction product with a high sensitivity. Heretofore, for measuring the β-D-galactosidase activity in the field of EIA, a method has been employed which comprises using chiefly a synthetic substrate and, as a means for terminating the β-D-galactosidase catalyzed reaction, shifting the pH value of the reaction mixture to the alkaline side which strictly deviates from the optimal pH range for β-D-galactosidase. However, although shifting the pH for terminating the enzyme reaction was operatively simple, this procedure could not completely inhibit β-D-galactosidase. If therefore the amount of the reaction product cannot be measured immediatley after the termination of the β-D-galactosidase catalyzed reaction, the action of β-D-galactosidase upon the remaining substrate in the reaction mixture gradually proceeds and accordingly it is difficult to determine the accurate amount of the reaction product. Furthermore the measured results frequently are scattering over a broad range. Especially, with the increase in reaction temperature, the phenomenon of this scattering in measured values is more pronounced.

From "Steroids", Vol. 37, March 1981, pages 303 to 314 and from R. J. Mayer, J. H. Walker "Immunochemical Methods in the Biological Sciences: Enzymes and Proteins", 1980, Academic Press, New York, page 52, it has been known that β-D-galactosidase is inhibited by a glycine-NaOH-buffer solution at a pH of 10 and 10.5, respectively.

According to Huber et al "Biochemistry", Vol. 18, no. 19, 1979, pages 4090 to 4095 $Mg^{+2}$ and $Mn^{+2}$-cations show the effect of activating β-galactosidase.

Since it has been known that amino acids having a chelating effect on metal ions, it could be expected that the activating action of some metal ions on β-galactosidase may be inhibited by adding a chelating agent to the reaction solution.

In recent years it has become a preferable operational step to use β-D-galactosidase in the insolubilized form when carrying out enzyme immuno assays. It has been found that insolubilized β-D-galactosidase may not be sufficiently inhibited either by shifting the pH of the reaction medium to the alkaline range or by using a chelating agent. This may be shown by the comparative tests included in the examples of this application.

In order to solve the above-mentioned problems in inhibiting the β-D-galactosidase activity, it has been the objective of the present invention to find a reliable method which allows the precise inhibition of the enzymatic activity of β-D-galactosidase in the insolubilized form.

Subject matter of the present invention is a method for inhibiting β-D-galactosidase by means of a chelating agent, which is characterized in that β-D-galactosidase in the insolubilized form is contacted with ethylenediaminetetraacetic acid, ethylenediamine, oxalic acid, oximes, acetyl acetone, pyrophosphoric acid, hydroxylamine, chelating polymers, metal adsorbing polymers or salts or mixtures thereof, at a pH of from 9 to 11.5.

According to this method, since the operation is simple and β-D-galactosidase activity may be determined without any restriction from the aspect of the measuring time, this method is extremely advantageous in practice. Of course, the conventional conditions and manners for measuring the reaction product of the β-D-galactosidase catalyzed reaction may be directly employed.

The chelating agent used in this invention is a polydentate ligand which forms a chelate compound by coordinating with a metal ion, and may be used as a salt, such as an alkali metal salt of e.g. sodium, potassium etc., a heavy metal salt of e.g. cobalt, copper etc., and the like. Specific examples thereof include ethylenediaminetetraacetic acid (hereinafter referred to as "EDTA"), ethylenediamine, oxalic acid, oxines, amino acids, acetylacetone, pyrophosphoric acid, hydroxylamine, chelating polymers, metal adsorbing polymers etc. Among those, EDTA is generally preferred because it has an extremely strong effect to inhibit β-D-galactosidase and it does not require handling as a poisonous chemical. In general, the chelating agent has 2 or more functional groups coordinating with a metal ion, and it is known that their main coordinating atoms, namely oxygen, nitrogen, and sulfur atoms, coordinate in such form as O,O-coordination, N,N-coordination, S,S-coordination, O,N-coordination, S,N-coordination or O,S-coordination to form a chelate ring. Any chelating agent having such various coordinations may be employed in the present invention. While the chelating agent in the present invention may be used alone as a single chelating agent, it is generally preferred to use it in combination with other adjuvants, such as a buffer, because the effect to inhibit β-D-galactosidase is further enhanced. On this occasion, the present invention may be practiced without being influenced by the components, composition and liquid properties of the buffer used in combination with the chelating agent or without being restricted by the source of β-D-galactosidase or the kind of the reaction product to be measured. Further, on practicising the present invention, β-D-galactosidase may be in either free form or insolubilized form, and the means for measuring the reaction product from the enzyme substrate by β-D-galactosidase may be any of e.g. colorimetry, spectrometry,

**0 075 293**

fluorophotometry, emission spectrometry etc. The amount of the chelating agent to be used in the present invention is not particularly restricted because even an extremely small amount can satisfactorily exhibit the effect to inhibit β-D-galactosidase, but it is desired that the chelating agent is present in an amount of 1 mM or more, taking into consideration the relationship with the amount of β-D-galactosidase to be used.

β-D-galactosidase and the chelating agent are brought into contact with each other at an alkaline pH range, i.e., pH 9—11.5, preferably pH 10—10.5.

The present invention is more particularly described by the following examples.

Example 1

Inhibitory effects of various chelating agents on insolubilized β-D-galactosidase

According to the method by Kato et al (Kanefusa Kato et al, *Kagaku to Seibutsu, 14* (No. 11) 737, *14* (No. 12) 817 (1976)), glass beads (6 mmφ×6 mm) having TSH (thyroid stimulating hormone) antibody insolubilized thereon and a TSH-enzyme complex labelled with β-D-galactosidase derived from Echerichia coli were prepared. One of the above beads and 0.01 ml of the diluted solution of the above TSH-enzyme complex were added to 3 ml of buffer solution A, prepared by the method of Kato et al, and incubated statically at 38°C overnight. Thereafter, the bead was washed with 1 ml of buffer solution A three times, transferred to another test tube containing 0.3 ml of the same buffer solution A, then 0.05 ml of $3\times10^{-4}$ solution of the enzyme substrate, 4 - methylumbelliferyl - β - D - galactoside was added, and the mixture was incubated at 37°C for one hour. After one hour of incubation, 2.5 ml of a 0.1 M glycine-NaOH buffer (pH 10.3) or a buffer prepared by adding 1 mM of one of the various reagents given below to the above buffer and readjusting to pH 10.3 was added, and the fluorescence intensity of 4-methylumbelliferone produced was measured using an excitation wavelength of 360 nm and at an emission wavelength of 450 nm (the first measurement). Thereafter, the above respective mixtures after the first measurement were allowed to stand at room temperature for 24 hours, and again the fluorescence intensity was measured as above (the second measurement).

The results of the second measurement expressed relative to the fluorescence intensity obtained in the first measurement taken as 100 are as follows:

| Reagent added to 0.1 M glycine-NaOH buffer (pH 10.3) | Results in the second measurement (fluorescence intensity) |
| --- | --- |
| None | 116.7 |
| Monoiodoacetic acid | 111.8 |
| D-Galactonic acid-γ-lactone | 112.5 |
| Isopropanol | 119.4 |
| EDTA Na salt | 101.0 |
| Ethylenediamine | 99.3 |
| Oxalic acid | 100.1 |
| Pyrophosphoric acid | 98.1 |
| Hydroxylamine | 100.4 |
| EDTA Cu salt | 99.8 |

Evidently, the inhibitory effects of the various chelating agents on the enzymatic activity of insolubilized β-D-galactosidase were also observed significantly.

Example 2

Inhibitory effect of EDTA on insolubilized β-D-galactosidase

Using commercially available α-fetoprotein measuring kits and immunoglobulin E measuring kits applying the EIA procedure in which β-D-galactosidase derived from Echerichia coli was used as the enzyme for labelling the antibody, the β-D-galactosidase activity insolubilized on glass beads or polystyrene beads was measured employing o - nitrophenyl - β - D - galactoside as the enzyme substrate according to the following procedures. To each measuring kit was added a 2 mM solution of p - nitrophenyl - β - D - galactoside and the reaction mixtures were incubated at 37°C. After one hour of incubation, borate buffer containing 1 mM EDTA (pH 9.8) was added to each mixture, and the amount of p-nitrophenol produced was measured by the absorption at a wavelength of 420 nm (referred to as the first

4

measurement). Thereafter, the above respective mixtures after the first measurement were kept overnight under the reaction temperature indicated above, and again the absorption at the same wavelength was measured (the second measurement).

The results are summarized in the following table.

| Kind of measuring kit | Borate buffer in the first measurement | Results of the second measurement (relative absorption at a wavelength of 420 nm) |
|---|---|---|
| α-Fetoprotein | D | 115.3 |
| α-Fetoprotein | E | 101.8 |
| Immunoglobulin E | D | 109.6 |
| Immunoglobulin E | E | 98.9 |

Evidently, the inhibitory effect of EDTA on β-D-galactosidase was extremely great regardless of its insolubilized condition and even when the measuring method of the insolubilized β-D-galactosidase activity was the visible spectrometry.

Example 3

Relationship between the inhibitory effect of EDTA on β-D-galactosidase and the pH

Similarly as in Example 1, glass beads having the TSH antibody insolubilized thereon were contacted with the TSH-enzyme complex labelled with β-D-galactosidase derived from Echerichia coli, to prepare beads having β-D-galactosidase insolubilized on their surface. Thereafter, to test tubes, each containing 0.30 ml of a 0.01 M phosphate buffer (pH 7.3) containing 0.1% bovine serum albumin, 0.1% $NaN_3$, 0.1 M NaCl and 10 mM $MgCl_2$, were transferred the above beads with insolubilized β-D-galactosidase, and, after adding 0.05 ml of the enzyme substrate solution employed in Example 1, each mixture was incubated statically at 37°C. After one hour of incubation, 2.5 ml of a 0.1 M glycine-NaOH buffer containing 1 mM EDTA and adjusted to pH 7.3, pH 8.5, pH 9.4 and pH 10.3, respectively, was added, and the fluorescence intensity of 4-methylumbelliferone produced was measured according to the procedures in Example 1 (the first measurement). The above respective mixtures after the first measurement were kept at 37°C for 3 hours, and again the fluorescence intensity was measured (the second measurement). The results obtained in the second measurement expressed relative to the fluoresence intensity in each section obtained in the first measurement taken as 100 are given below.

| pH of 0.1 M glycine-NaOH buffer containing 1 mM EDTA | Results in the second measurement (fluorescence intensity) |
|---|---|
| 7.3 | 357.9 |
| 8.5 | 124.5 |
| 9.4 | 101.8 |
| 10.3 | 99.3 |

Evidently, the inhibitory effect of EDTA on β-D-galactosidase significantly manifested in the strongly alkaline pH range greatly deviating from pH 7.3, which is the optimal pH range for β-D-galactosidase.

**Claims**

1. A method for inhibiting β-D-galactosidase by means of a chelating agent, characterized in that β-D-galactosidase in the insolubilized form is contacted with ethylenediaminetetraacetic acid, ethylenediamine, oxalic acid, oximes, acetylacetone, pyrophosphoric acid, hydroxylamine, chelating polymers, metal adsorbing polymers or salts or mixtures thereof, at a pH of from 9 to 11.5.

2. A method according to claim 1, wherein the chelating agent is ethylenediaminetetraacetic acid or a salt thereof.

3. A method according to claim 1, wherein the salt is an alkali metal salt or a heavy metal salt.

4. A method according to any one of claims 1 to 3, wherein the chelating agent is used in an amount of 1 mM or more.

**0 075 293**

5. A method according to any one of claims 1 to 4, wherein the β-D-galactosidase and the chelating agent are brought into contact with each other at a pH of from 10 to 10.5.

**Patentansprüche**

1. Verfahren zum Hemmen von β-D-Galactosidase mit Hilfe eines Chelatbildners, dadurch gekennzeichnet, daß β-D-Galactosidase in unlöslich gemachter Form bei einem pH-Wert von 9 bis 11,5 Mit Ethylenediamintetraessigsäure, Ethylendiamin, Oxalsäure, Oximen, Acetylaceton, Pyrophosphorsäure, Hydroxylamin, chelatbildenden Polymeren, Metall-adsorbierenden Polymeren oder Salzen oder Gemischen dieser in Berührung gebracht wird.

2. Verfahren nach Anspruch 1, bei dem der Chelatbildner Ethylendiamintetraessigsäure oder ein Salz davon ist.

3. Verfahren nach Anspruch 1, bei dem das Salz ein Alkalimetallsalz oder Schwermetallsalz ist.

4. Verfahren nach einem der Ansprüche 1 bis 3, bei dem der Chelatbildner in einer Menge von 1 mM oder mehr verwendet wird.

5. Verfahren nach einem der Ansprüche 1 bis 4, bei dem die β-D-Galactosidase und der Chelatbildner bei einem pH von 10 bis 10,5 miteinander in Berührung gebracht werden.

**Revendications**

1. Procédé pour inhiber la β-D-galactosidase au moyen d'un agent chélatant, caractérisé lorsque l'on met en contact la β-D-galactosidase sous forme insolubilisée avec l'acide éthylènediaminetétraacétique, l'éthylènediamine, l'acide oxalique, les oximes, l'acétylacétone, l'acide pyrophosphorique, l'hydroxylamine, les polymères chélatants, les polymères adsorbant les métaux ou leurs sels ou leurs mélanges à un pH de 9 à 11,5.

2. Procédé selon la revendication 1, dans lequel l'agent chélatant est l'acide éthylènediaminetétraacétique ou un de ses sels.

3. Procédé selon la revendication 1, dans lequel le sel est un sel de métal alcalin ou un sel de métal lourd.

4. Procédé selon l'une quelconque des revendications 1 à 3, dans lequel l'agent chélatant est utilisé en quantité de 1 mM ou plus.

5. Procédé selon l'une quelconque des revendications 1 à 4, dans lequel la β-D-galactosidase et l'agent chélatant sont mis en contact l'un avec l'autre à un pH de 10 à 10,5.

6